(19) Europäisches Patentamt
European Patent Office
Office européen des brevets

(11) EP 4 649 977 A1

(12) EUROPEAN PATENT APPLICATION

(43) Date of publication:
**19.11.2025 Bulletin 2025/47**

(21) Application number: **24175732.7**

(22) Date of filing: **14.05.2024**

(51) International Patent Classification (IPC):
***A61M 5/31*** *(2006.01)*

(52) Cooperative Patent Classification (CPC):
**A61M 5/3129;** A61M 2005/3131; A61M 2205/0222; A61M 2205/0238; A61M 2207/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **SCHOTT Pharma Schweiz AG**
**9001 St. Gallen (CH)**

(72) Inventors:
- **BRÄNDLE, Stephan**
  **9001 St. Gallen (CH)**
- **WÜST, Martin**
  **9001 St. Gallen (CH)**

(74) Representative: **Schott Corporate IP**
**Hattenbergstraße 10**
**55122 Mainz (DE)**

# (54) METHOD OF MAKING A COATED CONTAINER

(57) This disclosure relates to a method of making a coated container, e.g. a syringe. This disclosure particularly relates to method of making a container with a lubricant coating on an inner surface. This disclosure also relates to coated containers.

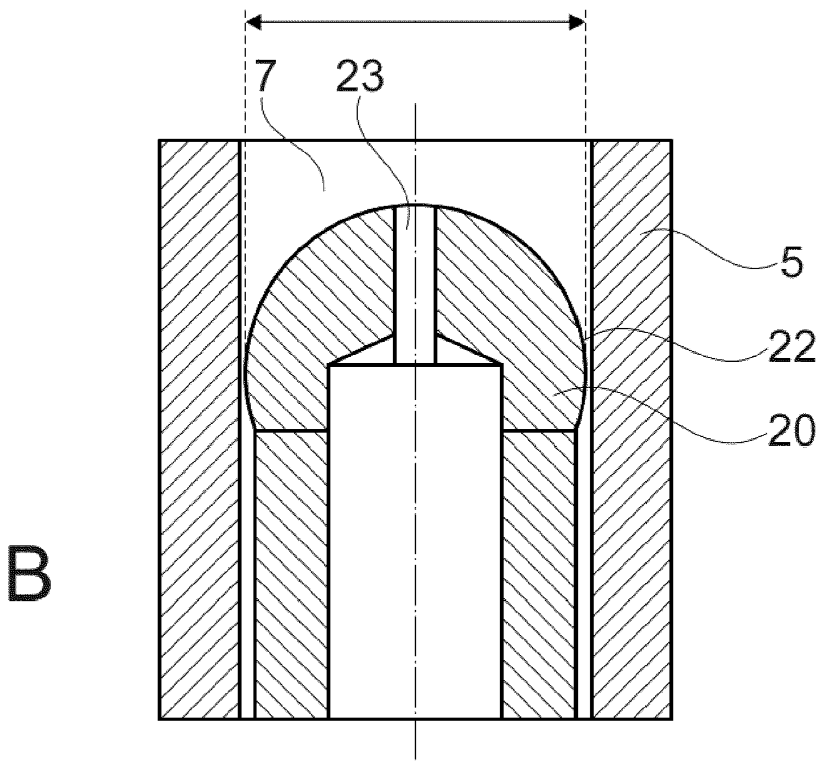


Fig. 3

EP 4 649 977 A1

## Description

This disclosure relates to a method of making a coated container, e.g. a syringe. This disclosure particularly relates to method of making a container with a lubricant coating on an inner surface. This disclosure also relates to coated containers.

## Background of the Disclosure

Pharmaceutical containers must comply with very strict requirements, including resistance to breakage and leakage. These containers must also be optically flawless in order not to disturb the patient's or physician's confidence in the quality of the pharmaceutical composition contained in the container.

Some pharmaceutical containers such as syringes require smooth movement of a stopper to expel the container's contents. It may be necessary to apply a coating to an inner surface of the pharmaceutical container to achieve this goal. It is desirable that any coating applied to a contact surface of container and stopper improves smooth movement, and that any inhomogeneities do not affect this smooth movement of the stopper. Stopper movement should be very homogeneous, i.e. it should not change abruptly along the path of stopper movement. Abrupt changes in gliding force would otherwise impede an easy and painless administration of a pharmaceutical composition from the container.

Pharmaceutical containers are needed in huge numbers. To facilitate production of such containers in the millions or even billions of units, simple and robust production technology is key. For example, pre-filled syringes are a type of pharmaceutical container that has gained much attention in recent years. Market research indicates that the pre-filled syringes market is expanding significantly. The demand for pre-filled syringes is driven by various factors, including the increased production of vaccines and the adoption of self-administered biologics for chronic conditions. According to a recent study, the sales of pre-filled syringes are expected to grow at about 9% per year until 2027 to a value of about 9 billion USD.

There is a need for pharmaceutical containers and methods of production that meet one or more of the objectives discussed above.

## Brief Summary of the Disclosure

In a first aspect, this disclosure relates to a method of making a coated container, comprising

- providing a container comprising a hollow cylindrical body having a wall surrounding a lumen, the hollow cylindrical body having at least one opening,
- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the deposition area during the depositing step.

Prior art methods of making coated containers include spray coating methods. Spray coating methods are extremely difficult to control so that a perfectly homogenous coating layer is not achieved. Other methods rely on a semi-spherical application body inserted into the container such that it contacts the inner walls of the container. After insertion, a liquid coating composition is applied to the application body such that it distributes the coating composition as the application body is retracted from the container.

In contrast to the "press-fit" coating process, the method of this disclosure features a circumferential gap between the application body and the deposition area during the depositing step, particularly without the application body contacting the inner surface of the hollow cylindrical body. There is no press-fit, i.e. the application body is not crammed into the hollow cylindrical body but leaves a gap. This gap allows for the coating composition, which is applied to the application body, to touch or contact at least a part of the inner surface of the wall and the application body, thereby bridging the gap between the application body and the inner surface, forming a so-called capillary bridge.

This capillary bridge facilitates a completely different way of coating the container compared to the press-fit. The

inventors hypothesize that in the former process the coating composition runs down the inner surface of the hollow cylindrical body as a free running film. To the contrary, the capillary bridge technique pulls the silicone cocktail over the surface like a blanket over a bed. Hence, only a thin liquid layer remains on the inner surface while most of the fluid is dragged away with the moving application body. Due to the drag force, there is constant stress along the liquid film. This certainly reduces any tendencies for larger accumulation of coating composition compared to press-fit prepared films. Without wishing to be bound by this theory, the inventors believe that this contributes to the superior coating qualities achievable with this capillary bridge technology.

In a second aspect, this disclosure relates to a coated container, optionally obtained or obtainable by the method of the first aspect, comprising a hollow cylindrical body having a wall surrounding a lumen, the hollow cylindrical body having at least one opening, wherein at least a part of an inner surface of the wall comprises a coating, wherein

- an average coating thickness is from 100 to 3000 nm, and
- wherein a total area of excessive coating thickness is less than 10% of the coating area and an excessive coating thickness is defined as an area exhibiting a coating thickness of more than twice the average coating thickness.

As discussed above, the method of this disclosure allows for production of coated containers having superior film properties. The coatings have a low variety of coating thicknesses, i.e. the proportions of areas with excessive thickness or very low thickness are small. This will achieve good and uniform gliding properties of a stopper used in the pharmaceutical container, particularly in pre-filled syringes. Uniform film thickness will also reduce the tendency of the coating to be rubbed off the inner surface by stopper movement because local stress maxima are reduced. Thereby, the coating technique used in this disclosure will contribute to reducing potential contaminations of pharmaceutical preparations in the container, particularly in case of long storage times in pre-filled syringes.

Brief Description of the Drawings

Figure 1 shows an exemplary pharmaceutical container.

Figure 2 is a schematic drawing of some of the method steps of a method of this disclosure.

Figure 3A is a schematic drawing of an application body inserted in a hollow cylindrical body in press-fit mode.

Figure 3B is a schematic drawing of an application body inserted in a hollow cylindrical body with a circumferential gap in accordance with this disclosure.

Figure 4A illustrates a thickness distribution of a coating applied by the press-fit technique.

Figure 4B illustrates a thickness distribution of a coating applied using the capillary bridge technique of this disclosure.

Figure 5A is a schematic drawing of an application body according to this disclosure.

Figure 5B is an illustration of a capillary bridge geometry obtainable in the method of this disclosure.

Detailed Description of the Disclosure

**Method**

In an embodiment, this disclosure relates to a method of making a coated container, comprising

- providing a container comprising a hollow cylindrical body having a wall surrounding a lumen, the hollow cylindrical body having at least one opening,
- inserting an application body into the lumen through the opening,
- applying a coating composition to the application body such that the coating composition contacts at least a section of an inner surface of the wall,

- depositing the coating composition on a deposition area of the inner surface of the wall by moving the application body relative to the hollow cylindrical body,

- retracting the application body from the lumen through the opening,

wherein the size of the application body is such that a circumferential gap is present between the application body and the inner surface during the depositing step.

The pharmaceutical container and/or the wall may be partially or entirely made of a material suitable for pharmaceutical primary packaging. Suitable material includes glass or polymers. The glass may be a silicate glass such as a borosilicate glass. The polymers may be amorphous polymers. Transparent polymers are preferred. Suitable polymers may be selected from the group consisting of cyclic olefin copolymers (COC), cyclic olefin polymers (COP), polyethylene terephthalate (PET), polycarbonate (PC), polypropylene (PP), and methyl methacrylate acrylonitrile butadiene styrene polymer (MABS). These polymers have the advantage of low density, high transparency, low birefringence, extremely low water absorption, excellent water vapor barrier properties, high rigidity, strength and hardness, excellent biocompatibility, very good resistance to acids and alkalis, and very good melt processability.

The wall and/or the pharmaceutical container may be made of polymer. Optionally, a polymer is chosen that has low density compared to glass, such as a density of from 0.90 to 1.20 g/cm$^3$, or >1.00 to 1.10 g/cm$^3$. Transport costs can be reduced if low density material is used. The density may be determined using the method described in ISO 1183-1:2013-04. The wall thickness may be from 1 mm to 2.5 mm or from 1.2 mm to 2 mm or from 1.3 mm to 1.9 mm.

In embodiments, the application body is inserted and retracted through the same opening. In the case of the container being a syringe, this will typically be the opening on the side of the flange. In certain embodiments, the opening through which the application body is retracted is facing downwards. Typically, the direction of movement in the depositing step will be same as the direction of retraction in the retracting step of the method. Thus, in some embodiments, at least a part coating composition is deposited on the deposition area by a downward movement of the application body. During this movement, the capillary bridge bridges the gap between the application body and the inner surface of the wall, thereby depositing the coating composition very evenly on the deposition area. In this disclosure, the "deposition area" is the part of the inner surface of the wall where a coating is desired and/or formed during the coating process by depositing the coating composition. It should be understood that inserting, moving and retracting the application body include the case that the application body is stationary, and the container is moved, or both application body and container move. In other words, the indicated movements are to be understood as relative movements. In some embodiments, more than 80% (vol/vol) of the coating composition, or substantially all of the coating composition is deposited on the deposition area by a downward movement of the application body.

Insertion of the application body is completed when the application body is in its initial position. The "initial position" is the position within the cylindrical body where the step of applying the coating composition to the application body takes place. If the deposition of coating composition is performed in a drawing movement, i.e. in the opposite direction of insertion, the initial position will be closer to the opposite end of the deposition area compared to the opening through which insertion of the application body was performed. If the deposition of coating composition is performed in a pushing movement, i.e. in the direction of insertion, the initial position will be closer to the end of the deposition area adjacent or close to the opening through which insertion of the application body was performed. It was found that deposition in a drawing movement has benefits over the pushing movement in that a more homogenous coating is obtained.

Optionally, the gap may be an annular gap with an essentially round cross-section. Generally, it will be easier to obtain a homogenous coating with essentially round cross-sections of both application body and hollow cylindrical body. In embodiments, the application body has a circumferential portion of closest distance to the inner surface of the wall when present in the hollow cylindrical body. This portion can be termed the "equator" of the application body, regardless of whether the application body is spherical or not. The application body may have spherical, hemispherical, conical or any other shape suitable for achieving a capillary bridge as discussed herein. Generally, it is desirable that the application body has an essentially spherical, hemispherical or conical shape on the side of the equator where the coating composition is applied to the application body. In an embodiment, the coating composition is applied to the upward-facing side of the application body so that the coating composition can flow downwards in the direction of the gap, where it may form a capillary bridge. In an embodiment, the application body has a shape of increasing diameter from the upwards-facing side in the direction of its equator.

The size of the circumferential gap may be characterized by a distance D between the application body and the deposition area during the depositing step. Distance D is defined as the average distance between the application body and the deposition area during the depositing step. The "average distance" is calculated by subtracting the average diameter of the application body $D_{AB}$ from the average inner diameter of the hollow cylindrical body $D_{ID}$, and division by 2 $(D = \frac{D_{ID} - D_{AB}}{2})$. $D_{AB}$ is the arithmetic mean of the largest and the smallest outer diameter around the outer

circumference of the application body. $D_{ID}$ is the arithmetic mean of the largest and the smallest inner diameter around the inner circumference of the hollow cylindrical body. In each case, if $D_{AB}$ or $D_{ID}$, respectively, are not the same along the longitudinal axis of the container, $D_{AB}$ and $D_{ID}$ are determined at a cross-section of application body or hollow cylindrical body, respectively, were the largest values for $D_{AB}$ or $D_{ID}$ are measured with the proviso that $D_{ID}$ should correspond to a section of the inner diameter corresponding to the deposition area. Optionally, the size of the circumferential gap is characterized by a distance D of less than 0.10 mm. Optionally, distance D is at least 0.005 mm, at least 0.01 mm, at least 0.02 mm or at least 0.03 mm. It may range up to 0.10 mm, up to 0.08 mm, up to 0.06 mm or up to 0.04 mm. For example, it may range from 0.005 to 0.10 mm, from 0.01 to 0.08 mm, or from 0.02 mm to 0.04 mm.

A relative gap size may be defined as $D/D_{AB}$. In an embodiment, $D/D_{AB}$ is from 0.0005 to 0.02, from 0.001 to 0.01, from 0.0015 to 0.006. Optionally, $D/D_{AB}$ is at least 0.0005, at least 0.001, at least 0.0015, or at least 0.002. In certain embodiments, $D/D_{AB}$ is up to 0.02, up to 0.01, up to 0.08, up to 0.06, or up to 0.04.

It is useful to choose a container with a hollow cylindrical body meeting strict geometrical parameters. In an embodiment, the cylindrical body has a total inner diameter variation in the deposition area of at most 2D, at most 1.5D, or at most D. Optionally, the total inner diameter variation is less than 0.10 mm, less than 0.08 mm, less than 0.06 mm or less than 0.04 mm. The smaller the total inner diameter variation, the better. However, in some embodiments it may not be economically feasible to provide containers with extremely small total inner diameter variations. Thus, in an embodiment, the total inner diameter variation may be at least 0.0001 mm, at least 0.001 mm, or at least 0.01 mm. For example, the total inner diameter variation may range from 0.0001 mm to <0.10 mm, from 0.001 mm to <0.08 mm, or from 0.01 mm to <0.04 mm.

To facilitate a very homogeneous coating, the application body should meet stringent quality criteria as well. For example, the application body may have a total outer diameter variation at its equator of at most 2D, at most 1.5D, or at most D. Optionally, the total outer diameter variation is less than 0.20 mm, less than 0.15 mm, less than 0.10 mm or less than 0.04 mm. The smaller the total outer diameter variation, the better. However, in some embodiments it may not be economically feasible to provide application bodies with extremely small total outer diameter variations. Thus, in an embodiment, the total outer diameter variation may be at least 0.0001 mm, at least 0.001 mm, or at least 0.003 mm. For example, the total outer diameter variation may range from 0.0001 mm to <0.20 mm, from 0.001 mm to <0.15 mm, or from 0.003 mm to <0.04 mm.

In an embodiment, the application body comprises or consists of a polymeric material. The application body may be coated or uncoated. Generally, the material of the application body is not limited as long as it is available in sufficient dimensional accuracy (see above). In an embodiment, at least a part of a surface of the application body that comes into contact with the coating composition during the method of this disclosure is made of or coated with a fluorinated polymer, such as PTFE.

In an embodiment, the application body and/or a coating on the application body comprises a resin, such as a fluorinated polymer such as a polymer selected from the group consisting of polytetrafluoroethylene (PTFE), densified expanded polytetrafluoroethylene (ePTFE), tetrafluoroethylene (TFE), tetrafluoroethylene-perfluoroethylene copolymer, tetrafluoroethylene-hexafluoropropylene copolymer, tetrafluoroethylene-ethylene copolymer, trichlorotrifluoroethylene, poly-vinylidene fluoride, polyvinyl fluoride, perfluoropropylvinylether, perfluoroalkoxy polymers, as well as copolymers, blends and combinations thereof. The coating may also be formed by layers comprising polyethylene, polypropylene, polyparaxylxylene, polylactic acid, as well as copolymers, blends and combinations thereof. A PTFE coating is a coating option. These coatings reduce the coefficient of friction of the body's surface on the inner surface of the hollow cylindrical body.

At least a part of the surface of the application body, such as the part of the surface that contacts the capillary bridge, may have a water contact angle of at least 100°, or even at least 110°. This surface may be superhydrophobic.

The inner surface of the hollow cylindrical body may have a surface energy of up to 45 mN/m, up to 40 mN/m, or up to 35 mN/m. Optionally, the surface energy is at least 15 mN/m, at least 20 mN/m, or at least 25 mN/m. For example, the surface energy of the inner surface may range from 15 mN/m to 45 mN/m, from 20 mN/m to 40 mN/m, or from 25 mN/m to 35 mN/m. Preferably, the surface energy of the inner surface is higher than the surface energy of the surface of the application body where the capillary bridge is formed. For example, the surface energy of the application body may be up to 25 mN/m, or up to 20 mN/m. Optionally, it may be at least 10 mN/m or at least 15 mN/m. In an embodiment, the surface energy of the application body where the capillary bridge is formed ranges from 10 mN/m to 25 mN/m or from 15 mN/m to 20 mN/m. In an embodiment, the surface energy of the inner surface of the hollow cylindrical body exceeds the surface energy of the application body by at least 40%, at least 50% or at least 60%. The surface energy can be measured indirectly by calculating the value with the Owens-Wendt-Rabel-Kaelble (OWRK) method from contact angle measurements according to DIN 55660-2:2011-12, chapter 6.2. The surface energy of the coating composition may be less than the surface energy of the application body and/or the inner surface of the hollow cylindrical body. Optionally, the surface energy of the coating composition is less than 20 mN/m, less than 17 mN/m or less than 15 mN/m. In an embodiment, the surface energy of the coating composition is at least 5 mN/m, at least 8 mN/m or at least 10 mN/m. For example, the surface energy of the coating composition may range from 5 mN/m to 20 mN/m, from 8 mN/m to 17 mN/m, or from 10 mN/m to 15 mN/m. Suitable

surface energies contribute to forming a capillary bridge.

In an embodiment, the application body comprises one or more ducts suitable for delivering the coating composition through the application body. One or more ducts are useful as it will not be necessary to place a volume of the coating composition within the hollow cylindrical body prior to insertion of the application body. Instead, the application body can be inserted and placed properly within the lumen before the coating composition is applied. In an embodiment, one or more ducts end close to or at the tip of the application body. The tip of the application body is usually the topmost part of it when the coating composition is applied. From there, the coating composition can flow towards the circumferential gap and bridge the gap, forming a capillary bridge. Accordingly, the step of applying a coating composition to the application body may comprise delivering the coating composition through one or more ducts. Typically, the one or more ducts will end above the equator of the application body, allowing the coating composition to flow downwards on the application body. Applying the coating composition to the application body may comprise delivering the coating composition to a topmost section of the application body, allowing the coating composition to flow downwards on the application body. In an embodiment, the application body comprises at least two, at least three, at least four, or at least six ducts. Optionally, the number of ducts may range up to 24, up to 20, up to 16, or up to 12. For example, the application body may comprise from 1 to 24, from 2 to 20, from 4 to 16, or from 6 to 12 ducts. The ducts may be arranged over the application body in a manner that supports homogenous distribution of the coating composition.

Applying a coating composition to the application body may comprise delivering the coating composition to a section of the application body, and allowing the coating composition to build a capillary bridge between the application body and the inner surface of the wall.

As discussed above, the coating composition is applied to the application body such that the coating composition contacts at least a section of an inner surface of the wall. This requires the coating composition to be close enough to the inner surface of the wall to span the circumferential gap. In an embodiment, the size of the circumferential gap is such that the coating composition forms a capillary bridge spanning the circumferential gap during the depositing step. If the size of the gap is small enough to allow for a capillary bridge during the deposition step, in particular during the whole of the deposition step, a very uniform coating can be obtained. On the other hand, if the size of the gap is too small, the application body might inadvertently touch the inner surface in the deposition area, disturbing the uniform deposition of the coating composition.

In an embodiment, the volume of coating composition initially applied to the application body is sufficient to bridge the gap throughout the depositing step. In another embodiment, one or more further volume portions of coating compositions are applied to the application body during the depositing step, e.g. replenishing the volume of coating composition so that the capillary bridge will remain intact throughout the depositing step.

In an embodiment, the total volume of coating composition applied to the application body initially and optionally during the depositing step is at least 2 μl, at least 5 μl, at least 10 μl, at least 25 μl, or at least 50 μl. Optionally, this volume may be up to 200 μl, up to 100 μl or up to 90 μl. Of course, the exact volume of coating composition will depend on the deposition area and desired thickness of the coating. For example, the total volume of coating composition may be from 2 μl to 200 μl, or from 5 μl to 100 μl.

In an embodiment, a rate of movement of the application body during depositing the coating composition is not more than a flow rate of the coating composition in the direction of movement of the application body. As mentioned above, during depositing the coating composition, the application body moves relative to the hollow cylindrical body. During this step, the capillary bridge should remain intact in order to achieve a most homogeneous result. If the application body moves too quickly, a significant portion of coating composition may revert to a free flowing motion with the risk of rivulets forming on the inner surface. These rivulets would remain on the inner surface as severe inhomogeneities. Optionally, the rate of movement of the application body is at least 2 mm/s, at least 4 mm/s, at least 5 mm/s, or at least 10 mm/s. In an embodiment, the rate of movement is up to 50 mm/s, up to 30 mm/s, up to 20 mm/s, or up to 15 mm/s. For example, the rate of movement of the application body ranges from 2 mm/s to 50 mm/s, from 5 mm/s to 30 mm/s, or from 10 mm/s to 15 mm/s.

In an embodiment, the method may further comprise the step of air flushing the container after depositing the coating composition on the deposition area. Air flushing can contribute to evaporation of a diluent in the coating composition and helps smoothen the coating.

Additionally or alternatively, the method may include the step of curing the coating composition after depositing the coating composition on the deposition area to obtain a cured coating. Curing can be used to cross-link and/or polymerize cross-linkable or polymerizable compounds of the coating composition to form a coating. Also, curing may contribute to evaporation of diluent from the coating composition.

The coating composition may be applied so as to obtain a desired coating thickness. The coating may have an average thickness of 100 nm or more, 200 nm or more, or 300 nm or more, 400 nm or more, or 450 nm or more. Optionally, the coating may have an average thickness of up to 3000 nm, up to 2500 nm, up to 2000 nm, up to 1500 mm, up to 1000 mm, or up to 850 nm. A suitable coating thickness contributes to a tight seal at low temperatures. In embodiments, an average coating thickness may be from 100 nm to 3000 nm, from 200 nm to 2000 nm, or from 300 nm to 1500 nm, or from 400 nm to 1000 nm. Optionally, the indicated average coating thickness is present on at least 90%, at least 95% or at least 99% of the

coated area. In embodiments, the average coating thickness is larger than 400 nm, particularly at least 450 nm or at least 500 nm. Exemplary preferred ranges for the average coating thickness are from >400 nm to 1500 nm, from 450 nm to 1250 nm, or from 450 nm to 850 nm.

The coating composition may be deposited on at least 25%, or at least 50% of the inner surface of the hollow cylindrical body (area by area). The coating may have a beneficial effect on the gliding properties of a stopper on the inner surface of the hollow cylindrical body. Therefore, in some embodiments, the coating composition is deposited on at least 65%, or at least 85% of the inner surface of said hollow cylindrical body (area by area). Optionally, the coating composition is deposited on at least 90%, or essentially all of the inner surface of said hollow cylindrical body.

The "curing temperature" as used herein relates to the effective temperature of the coating for it to cure. The curing temperature is not the nominal temperature in an oven, which may be higher than the effective temperature of the coating during curing. Curing may include polymerizing polymerizable groups, such as polymerizable end groups. The coating may be cured at a curing temperature below 150°C, below 125°C, or below 110°C. Too high a curing temperature may yield a coating having a high brittleness. On the other hand, too low a curing temperature may not be sufficient for a good performance either. Thus, in embodiments, the curing temperature may be 50°C or more, 60°C or more, or 70°C or more. Notably, the curing temperature is the effective temperature at the coating composition. It must not be confused with a nominal oven temperature. The oven temperature may be much higher than the curing temperature because there may be insufficient time for the whole oven to equilibrate at the nominal temperature during curing time. Optionally, the coating may be cured at 50°C to below 150°C, from 60°C to below 125°C, or from 70°C to below 110°C. A preferred range is from 50°C to <110°C. In embodiments, curing does not involve the application of a plasma.

The curing temperature is held for a time sufficient to achieve the desired degree of curing. Optionally, the curing temperature may be held for at least 2 seconds, at least 3 seconds, at least 4 seconds or at least 5 seconds. In embodiments, the curing temperature is held for up to 300 seconds, up to 100 seconds, or up to 20 seconds.

After curing an average coating thickness may be from 100 to 3000 nm. Additionally or alternatively, after curing a total area of excessive coating thickness is less than 10% of the coating area and an excessive coating thickness is defined as an area exhibiting a coating thickness of more than twice, more than 1.50, or more than 1.20 the average coating thickness. After curing the coating may contain one or more silicon-organic polymers.

In an embodiment, the method includes the further step of obtaining a coated container of this disclosure. In an embodiment, the method includes the further step of obtaining a container having a coating as described in more detail below.

The coating composition may be cured at a curing temperature below 150°C, below 125°C, or below 110°C. Too high a curing temperature may yield a coating having high brittleness. On the other hand, too low a curing temperature may not be sufficient for a good mechanical resistance of the coating. Thus, in embodiments, the curing temperature may be 50°C or more, 60°C or more, or 70°C or more. Notably, the curing temperature is the effective temperature at the coating composition. It must not be confused with a nominal oven temperature. The oven temperature may be much higher than the curing temperature because there may be insufficient time for the whole oven to equilibrate at the nominal temperature during curing time. Optionally, the coating may be cured at 50°C to below 150°C, from 60°C to below 125°C, or from 70°C to below 110°C. A preferred range is from 50°C to <110°C.

In embodiments, the coating is obtainable or obtained by applying a coating composition as disclosed herein to at least parts of a surface of the container (e.g. an inner surface), and curing the coating composition on the surface.

This disclosure is not particularly limited to container volumes. In an embodiment, the hollow cylindrical body encloses a volume of at least 0.10 ml, at least 0.50 ml, or at least 1.00 ml. Optionally, the volume may be up to 1,000 ml, up to 200 ml, up to 100 ml, or up to 25 ml. In embodiment, the volume ranges from 0.1 ml to 1,000 ml, from 0.50 ml to 200 ml, or from 1.00 ml to 25 ml. In an embodiment, the hollow cylindrical body encloses a volume of less than 10.0 ml.

The hollow cylindrical body has a lumen surrounded by a wall, wherein the wall may have a wall thickness of at least 0.50 mm, at least 0.80 mm, or at least 1.00 mm. Optionally, the wall thickness may range up to 10.0 mm, up to 8.0 mm, up to 5.0 mm, or up to 4.0 mm. In embodiments, wall thickness is from 0.50 to 10.0 mm, from 0.80 mm to 8.0 mm, or from 1.00 mm to 4.00 mm. The term "wall thickness" as used herein describes the shortest distance between an inner surface and an outer surface of the hollow cylindrical body.

The term "outer diameter" as used herein refers to the maximum distance between two points on the outer surface of the hollow cylindrical body, wherein the two points are connected by a straight line, which is perpendicular to and intersects with the longitudinal axis of the hollow cylindrical body. The term "inner diameter" as used herein refers to the maximum distance between two points on the inner surface of the hollow cylindrical body, wherein the two points are connected by a straight line, which is perpendicular to and intersects with the longitudinal axis of the hollow cylindrical body.

In a first specific embodiment, the method comprises

- providing a container comprising a hollow cylindrical body having a wall surrounding a lumen, the hollow cylindrical body having at least one opening,

- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the inner surface during the depositing step,

wherein

• the wall is made of polymer, such as COC or COP;

• at least a part of the coating composition is deposited on the deposition area by a downward movement of the application body;

• the application body comprises one or more ducts suitable for delivering the coating composition through the application body; and

• the size of the circumferential gap is characterized by a distance D of less than 0.10 mm between the application body and the deposition area during the depositing step.

In a second specific embodiment, the method comprises

- providing a container comprising a hollow cylindrical body having a wall surrounding a lumen, the hollow cylindrical body having at least one opening,
- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the inner surface during the depositing step,

wherein

• the wall is made of polymer, such as COC or COP;

• at least a part of the coating composition is deposited on the deposition area by a downward movement of the application body;

• the application body comprises one or more ducts suitable for delivering the coating composition through the application body;

• the size of the circumferential gap is characterized by a distance D of less than 0.10 mm between the application body and the deposition area during the depositing step;

• the total volume of coating composition applied to the application body is from 5 μl to 100 μl; and

- a rate of movement of the application body during depositing the coating composition is not more than a flow rate of the coating composition in the direction of movement of the application body.

In a third specific embodiment, the method comprises

- providing a container comprising a hollow cylindrical body having a wall surrounding a lumen, the hollow cylindrical body having at least one opening,
- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the inner surface during the depositing step,

wherein

- the wall is made of polymer, such as COC or COP;
- at least a part of the coating composition is deposited on the deposition area by a downward movement of the application body;
- the application body comprises one or more ducts suitable for delivering the coating composition through the application body;
- the size of the circumferential gap is characterized by a distance D of less than 0.10 mm between the application body and the deposition area during the depositing step;
- the total volume of coating composition applied to the application body is from 5 µl to 100 µl; and
- a rate of movement of the application body during depositing the coating composition is not more than a flow rate of the coating composition in the direction of movement of the application body;
- the coating composition comprises polysiloxane structural units or compounds.

In a fourth specific embodiment, the method comprises

- providing a container comprising a hollow cylindrical body having a wall surrounding a lumen, the hollow cylindrical body having at least one opening,
- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the inner surface during the depositing step,

wherein

- the wall is made of polymer, such as COC or COP;
- at least a part of the coating composition is deposited on the deposition area by a downward movement of the application body;
- the application body comprises one or more ducts suitable for delivering the coating composition through the application body;
- the size of the circumferential gap is characterized by a distance D of less than 0.10 mm between the application body and the deposition area during the depositing step;
- the total volume of coating composition applied to the application body initially and optionally during the depositing step is from 5 µl to 100 µl;
- a rate of movement of the application body during depositing the coating composition is not more than a flow rate of the coating composition in the direction of movement of the application body;
- the coating composition comprises polysiloxane structural units or compounds; and
- the surface energy of the inner surface is higher than the surface energy of the surface of the application body where the capillary bridge is formed.

In a fifth specific embodiment, the method comprises

- providing a container comprising a hollow cylindrical body having a wall surrounding a lumen, the hollow cylindrical body having at least one opening,
- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the inner surface during the depositing step,

wherein

• the wall is made of glass;

• at least a part of the coating composition is deposited on the deposition area by a downward movement of the application body;

• the application body comprises one or more ducts suitable for delivering the coating composition through the application body; and

• the size of the circumferential gap is characterized by a distance D of less than 0.10 mm between the application body and the deposition area during the depositing step.

In a sixth specific embodiment, the method comprises

- providing a container comprising a hollow cylindrical body having a wall surrounding a lumen, the hollow cylindrical

body having at least one opening,

- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the inner surface during the depositing step,

wherein

• the wall is made of glass;

• at least a part of the coating composition is deposited on the deposition area by a downward movement of the application body;

• the application body comprises one or more ducts suitable for delivering the coating composition through the application body;

• the size of the circumferential gap is characterized by a distance D of less than 0.10 mm between the application body and the deposition area during the depositing step;

• the total volume of coating composition applied to the application body is from 5 µl to 100 µl; and

• a rate of movement of the application body during depositing the coating composition is not more than a flow rate of the coating composition in the direction of movement of the application body.

In a seventh specific embodiment, the method comprises

- providing a container comprising a hollow cylindrical body having a wall surrounding a lumen, the hollow cylindrical body having at least one opening,
- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the inner surface during the depositing step,

wherein

• the wall is made of glass;

• at least a part of the coating composition is deposited on the deposition area by a downward movement of the application body;

- the application body comprises one or more ducts suitable for delivering the coating composition through the application body;
- the size of the circumferential gap is characterized by a distance D of less than 0.10 mm between the application body and the deposition area during the depositing step;
- the total volume of coating composition applied to the application body is from 5 µl to 100 µl; and
- a rate of movement of the application body during depositing the coating composition is not more than a flow rate of the coating composition in the direction of movement of the application body;
- the coating composition comprises polysiloxane structural units or compounds.

In an eighth specific embodiment, the method comprises

- providing a container comprising a hollow cylindrical body having a wall surrounding a lumen, the hollow cylindrical body having at least one opening,
- inserting an application body into the lumen through the opening,
- applying a coating composition to the application body such that the coating composition contacts at least a section of an inner surface of the wall,
- depositing the coating composition on a deposition area of the inner surface of the wall by moving the application body relative to the hollow cylindrical body,
- retracting the application body from the lumen through the opening,

wherein the size of the application body is such that a circumferential gap is present between the application body and the inner surface during the depositing step,

wherein

- the wall is made of glass;
- at least a part of the coating composition is deposited on the deposition area by a downward movement of the application body;
- the application body comprises one or more ducts suitable for delivering the coating composition through the application body;
- the size of the circumferential gap is characterized by a distance D of less than 0.10 mm between the application body and the deposition area during the depositing step;
- the total volume of coating composition applied to the application body initially and optionally during the depositing step is from 5 µl to 100 µl;
- a rate of movement of the application body during depositing the coating composition is not more than a flow rate of the coating composition in the direction of movement of the application body;
- the coating composition comprises polysiloxane structural units or compounds; and
- the surface energy of the inner surface is higher than the surface energy of the surface of the application body where the capillary bridge is formed.

**Coating composition**

The coating composition may contain or consist of the ingredients necessary to obtain the coating described in

this disclosure. The coating composition may contain one or more silicon-organic substances, such as polymers or oligomers. For example, the coating composition may comprise polysiloxane structural units or compounds. The polysiloxane compounds of the coating composition may comprise cross-linkable polysiloxane compounds and/or non-cross-linkable polysiloxane compounds. In an embodiment, the polysiloxane compounds are polyalkylsiloxane compounds, such as polydialkylsiloxane compounds. Optionally, one or more of the alkyl groups in the polyalkylsiloxane or polydialkylsiloxane are independently selected from branched or unbranched C1 to C8 alkyl groups. Further, the coating composition may comprise a catalyst and/or a diluent.

A polysiloxane compound is considered "cross-linkable", if it comprises one or more (particularly two) groups that are polymerizable or cross-linkable under the curing conditions of this disclosure, particularly at a curing temperature below 150 °C and a curing time of less than 3000 seconds. A polysiloxane compound is considered "non-cross-linkable", if it does not comprise any chemical structures that are polymerizable or cross-linkable under the curing conditions of this disclosure, particularly at a curing temperature below 150 °C and a curing time of less than 3000 seconds.

In an embodiment, the coating composition comprises

- one or more cross-linkable polydialkylsiloxane compounds, and
- one or more non-cross-linkable polysiloxane compounds.

Optionally, the coating composition comprises

- one or more cross-linkable polydialkylsiloxane compounds,
- one or more non-cross-linkable polysiloxane compounds, and
- one or more cross-linking polysiloxane compounds.

Cross-linking polysiloxane compound

The cross-linking polysiloxane compound is suitable to react with the cross-linkable polysiloxane compound under the conditions of this disclosure (particularly at a curing temperature below 150 °C and a curing time of less than 3000 seconds), preferably in a hydrosilylation reaction, forming a polysiloxane network.

The cross-linking polysiloxane compound may comprise alkylsiloxane monomeric units, such as dialkylsiloxane monomeric units. Optionally, one or more of the alkyl groups of the monomers in the cross-linking polysiloxane compound are independently selected from branched or unbranched C1 to C8 alkyl groups. The alkyl groups may be linear alkyl groups. For example, the alkyl groups may be independently selected from methyl, ethyl, propyl, butyl, pentyl, hexyl, heptyl and octyl groups. Optionally, the alkyl groups are independently selected from methyl and ethyl.

In some embodiments, the cross-linking polysiloxane compound is a polysiloxane having Si-H groups. In an exemplary embodiment the cross-linking polysiloxane compound is a copolymer having dimethylsiloxane and methylhydrosiloxane monomer units. In this case, it has been found to be advantageous to use a copolymer having the following structure (m is an integer of 1 or more and n is an integer of 1 or more; n may be 2 or more):

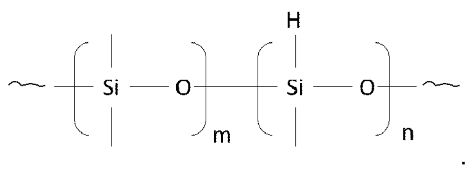

Optionally, the cross-linking polysiloxane is a copolymer, in particular having dialkylsiloxane and alkylhydrosiloxane monomeric units. The alkyl group in the alkylhydrosiloxane monomeric unit may be selected from branched or unbranched C1 to C8 alkyl groups. The alkyl groups may be linear alkyl groups. For example, the alkyl groups may be independently selected from methyl, ethyl, propyl, butyl, pentyl, hexyl, heptyl and octyl groups. Optionally, the alkyl groups are independently selected from methyl and ethyl.

The cross-linking polysiloxane may be present in the coating compositions at a concentration of from 0.10 to 1.50% by weight, from 0.10 to 1.00% by weight, or from 0.10 to 0.60% by weight. In embodiments, the concentration of cross-linking polysiloxane in the coating composition should not exceed 1.50% by weight, 1.00% by weight, or 0.60% by weight. A minimum amount of 0.10% by weight is preferred.

Non-cross-linkable polysiloxane compound

The coating composition may comprise more than one type of non-cross-linkable polysiloxane compounds, such as at least two types, or at least three types. The types may differ in their viscosities. In some embodiments, the coating comprises high viscosity non-cross-linkable polysiloxane compounds having a viscosity of more than 10,000 cSt, and/or low viscosity non-cross--linkable polysiloxane compounds having a viscosity of 10,000 cSt, or less. Viscosity of the polysiloxane compounds may be determined according to DIN EN ISO 3219:1993 using a coaxial-cylinder system at 23°C and a shear rate of 10 $s^{-1}$. Optionally, the high viscosity non-cross-linkable polysiloxane compounds have a viscosity of at least 15,000 cSt, and/or the low viscosity non-cross-linkable polysiloxane compounds have a viscosity of 5,000 cSt, or less.
A ratio of a weight amount of the low viscosity non-cross-linkable polysiloxane compounds and the high viscosity non-cross-linkable polysiloxane compounds ($mass_{high}$ : $mass_{low}$) may be at least 0.10, at least 0.50, at least 1.00, at least 1.50 or at least 2.00. In some embodiments, this ratio may range up to 5.00, up to 4.00 or up to 3.00. For example, the ratio of a weight amount of the low viscosity non-cross-linkable polysiloxane compounds and the high viscosity non-cross-linkable polysiloxane compounds may range from 0.10 to 5.00, from 0.50 to 4.00, or from 1.00 to 3.00.
Optionally, the low viscosity non-cross-linkable polysiloxane compounds have a weight average molecular weight of 1,200 to 30,000 g/mol, and/or the high viscosity non-cross-linkable polysiloxane compounds have a weight average molecular weight of 15,000 to 300,000 g/mol. In an embodiment, the high viscosity non-cross-linkable polysiloxane compounds have a weight average molecular weight of 32,000 to 210,000 g/mol, or from 100,000 to 150,000 g/mol. In an embodiment, the low viscosity non-cross-linkable polysiloxane compounds have a weight average molecular weight of 5,000 to 25,000 g/mol, or from 10,000 to 20,000 g/mol.
In embodiments, the low viscosity non-cross-linkable polysiloxane compounds have a weight average molecular weight of at least 1,200 g/mol, at least 5,000 g/mol, or at least 10,000 g/mol. The weight average molecular weight may range up to 30,000 g/mol, up to 25,000 g/mol, or up to 20,000 g/mol.
In embodiments, the high viscosity non-cross-linkable polysiloxane compounds have a weight average molecular weight of at least 15,000 g/mol, at least 32,000 g/mol, or at least 100,000 g/mol. The weight average molecular weight may range up to 300,000 g/mol, up to 210,000 g/mol, or up to 150,000 g/mol. Weight average molecular weights can be determined by gel permeation chromatography (GPC).
Polydimethylsiloxane is particularly suitable as non-cross-linkable polysiloxane compound.

Cross-linkable polysiloxane compound

The cross-linkable polysiloxane compound may be cross-linkable via one or more, preferably two, end groups. In particular, the end groups may have double bonds, making them available for a hydrosilylation reaction, e.g. under the conditions of this disclosure (particularly at a curing temperature below 150 °C and a curing time of less than 3000 seconds). The end groups may be selected from vinyl, acryl, methacryl, styrene, and combinations thereof.
In an embodiment, the cross-linkable polysiloxane compound and the cross-linking polysiloxane compound may form a hydrosilylation reaction product under the conditions of this disclosure (particularly at a curing temperature below 150 °C and a curing time of less than 3000 seconds). A suitable cross-linkable polysiloxane compound is a vinyl-polysiloxane compound. The cross-linking polysiloxane may cross-link the cross-linkable polysiloxane by reaction of a plurality of Si-H groups with vinyl groups of the cross-linkable polysiloxane. The reaction may be platinum catalyzed.
The cross-linkable polysiloxane compound may comprise alkylsiloxane monomeric units, such as dialkylsiloxane monomeric units. Optionally, one or more of the alkyl groups of the monomers in the cross-linkable polysiloxane compound are independently selected from branched or unbranched C1 to C8 alkyl groups. The alkyl groups may be linear alkyl groups. For example, the alkyl groups may be independently selected from methyl, ethyl, propyl, butyl, pentyl, hexyl, heptyl and octyl groups. Optionally, the alkyl groups are independently selected from methyl and ethyl.

Diluents

The coating composition may further comprise one or more diluents. A diluent within the context of this disclosure may be an Si-containing solvent in which the cross-linkable polysiloxane compound and the non-cross-linkable polysiloxane compound are soluble. To ensure good solubility of the polysiloxane compounds nonpolar solvents can be used as diluents. In this case, the use of silicon-organic compounds having at most 6 silicon atoms as diluent has been found to be useful.
Exemplary diluents are:

- cyclic silicones, such as: octamethylcyclotetrasiloxane, decamethylcyclopentasiloxane, dodecamethylcyclohexasiloxane, tetramethylcyclotetrasiloxane, pentamethylcyclopentasiloxane,

- hexamethyldisiloxane (HMDSO),
- octamethyltrisiloxane,
- decamethyltetrasiloxane.

A mixture, especially comprising one or more of the abovementioned substances, may also be used as diluent.

The coating composition is a liquid. In embodiments, the coating composition has a viscosity of from 1.0 to 50 mPas, from 2.0 to 25 mPas, or from 3.0 to 15 mPas. Optionally, the viscosity of the coating composition can be at least 1.0 mPas, at least 2.0 mPas, at least 3.0 mPas, or at least 3.5 mPas. For example, viscosity of the coating composition can range up to 50 mPas, up to 25 mPas, or up to 15 mPas. Viscosity of the coating composition may be determined according to DIN EN ISO 3219:1993 using a coaxial-cylinder system at 25°C and a shear rate of 16.8 s-1.

Catalyst/Inhibitor

The coating composition may further comprise a catalyst for the cross-linking reaction of compounds of the multicomponent system. A soluble platinum-containing catalyst, for example chloroplatinic acid, may be used. A Karstedt catalyst can be used.

In some embodiments, the coating composition comprises at least one inhibitor for preventing a spontaneous reaction of the composition. This facilitates handling of the composition up to application of the coating. The inhibitor can enter reversible complex formation with the catalyst, with the result that a spontaneous cross-linking reaction of the composition is prevented.

Coating compositions

In an embodiment, the coating composition comprises the high viscosity non-cross-linkable polysiloxane compound but not necessarily the low viscosity non-cross-linkable polysiloxane compound.

In an embodiment, the coating composition comprises the following components:

| |
|---|
| one or more cross-linkable polysiloxane compounds |
| one or more non-cross-linkable polysiloxane compounds |
| one or more cross-linking polysiloxane compounds |
| one or more catalysts |
| one or more diluents |

In an embodiment, the coating composition comprises the following components in percent by weight:

| | |
|---|---|
| one or more cross-linkable polysiloxane compounds | 3.0 to 20.0% |
| one or more non-cross-linkable polysiloxane | 2.0 to 25.0% |
| compounds | |
| one or more cross-linking polysiloxane compounds | 0.10 to 1.50% |
| one or more catalysts | 0.001 to 0.50% |
| one or more diluents | 55.0 to 92.0% |

In certain embodiments, a weight ratio of cross-linking polysiloxane compound and cross-linkable polysiloxane compound is at least 0.01, at least 0.015 or at least 0.02. Optionally, this ratio should not exceed 0.5, 0.4, 0.2 or 0.1. In some embodiments, this ratio ranges from 0.01 to 0.5, from 0.015 to 0.4, or from 0.02 to 0.2.

Optionally, a ratio of a weight amount of cross-linkable polysiloxane compounds and a weight amount of non-cross-linkable polysiloxane compounds in the coating composition is less than 3.00, less than 2.50, less than 1.80, or less than 1.20. The ratio of a weight amount of cross-linkable polysiloxane compounds and a weight amount of non-cross-linkable polysiloxane compounds in the coating may be at least 0.40, at least 0.60, or at least 0.70. In embodiments, this ratio ranges from 0.40 to 3.00, from 0.60 to 2.50, or from 0.70 to 1.80.

In an embodiment, the coating composition comprises the following components in percent by weight:

| | |
|---|---|
| one or more cross-linkable polysiloxane compounds | 3.0 to 20.0% |
| one or more non-cross-linkable polysiloxane compounds | 5.0 to 25.0% |
| one or more cross-linking polysiloxane compounds | 0.10 to 1.50% |
| one or more catalysts | up to 0.50%, e.g. 0.03 to 0.50% |
| one or more diluents | 55.0 to 92.0% |

In an embodiment, the coating composition comprises the following components in percent by weight:

| | |
|---|---|
| one or more cross-linkable polysiloxane compounds | 3.0 to 20.0% |
| one or more non-cross-linkable polysiloxane compounds | 8.0 to 25.0% |
| one or more cross-linking polysiloxane compounds | 0.10 to 1.50% |
| one or more catalysts | up to 0.50%, e.g. 0.03 to 0.50% |
| one or more diluents | 55.0 to 92.0% |

In an embodiment, the coating composition comprises the following components in percent by weight:

| | |
|---|---|
| one or more cross-linkable polysiloxane compounds | 7.5 to 15.0% |
| one or more non-cross-linkable polysiloxane compounds | 8.0 to 15.0% |
| one or more cross-linking polysiloxane compounds | 0.10 to 1.00% |
| one or more catalysts | up to 0.50%, e.g. 0.03 to 0.50% |
| one or more diluents | 55.0 to 82.0% |

In an embodiment, the coating composition comprises the following components in percent by weight:

| | |
|---|---|
| one or more cross-linkable polysiloxane compounds | 7.5 to 15.0% |
| one or more non-cross-linkable polysiloxane compounds | 2.0 to 8.0% |
| one or more cross-linking polysiloxane compounds | 0.10 to 1.00% |
| one or more catalysts | up to 0.50%, e.g. 0.03 to 0.50% |
| one or more diluents | 65.0 to 92.0% |

<u>More specific coating compositions</u>

The cross-linked polysiloxane structural units, the low viscosity non-cross-linked polysiloxane structural units, and/or the high viscosity non-cross-linked polysiloxane structural units, may comprise or consist of dialkylsiloxane monomeric units, in particular dimethylsiloxane monomeric units.

In some embodiments, the cross-linkable polysiloxane is a vinyl-functionalized polysiloxane and/or the cross-linking polysiloxane compound is a polysiloxane having Si-H groups. One exemplary embodiment contains, as cross-linkable polysiloxane compound, a vinyl-functionalized polydimethylsiloxane and/or, as cross-linking polysiloxane compound, a copolymer having dimethylsiloxane and methylhydrosiloxane monomer units. In this case, it has been found to be advantageous to use a copolymer having the following structure (m is an integer of 1 or more and n is an integer of 1 or more; n may be 2 or more):

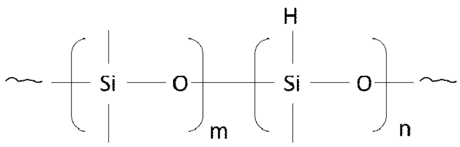

.

In a more specific variant, the coating composition comprises the following components:

| |
|---|
| a vinyl-functionalized polysiloxane as a cross-linkable polysiloxane compound |
| one or more non-cross-linkable polysiloxane compounds |
| a copolymer having dimethylsiloxane and methylhydrosiloxane monomer units as a cross-linking polysiloxane compound, |
| one or more Pt-containing catalysts |
| one or more diluents |

In a more specific variant, the coating composition comprises the following components in percent by weight:

| | |
|---|---|
| a vinyl-functionalized polysiloxane as a cross-linkable polysiloxane compound | 3.0 to 20.0% |
| one or more non-cross-linkable polysiloxane compounds | 2.0 to 25.0% |
| a copolymer having dimethylsiloxane and methylhydrosiloxane monomer units as a cross-linking polysiloxane compound, | 0.10 to 1.50% |
| one or more Pt-containing catalysts | up to 0.50%, e.g. 0.03 to 0.50% |
| one or more diluents | 55.0 to 92.0% |

In a more specific variant, the coating composition comprises the following components in percent by weight:

| | |
|---|---|
| a vinyl-functionalized polysiloxane as a cross-linkable polysiloxane compound | 3.0 to 20.0% |
| one or more non-cross-linkable polysiloxane compounds | 5.0 to 25.0% |
| a copolymer having dimethylsiloxane and methylhydrosiloxane monomer units as a cross-linking polysiloxane compound, | 0.10 to 1.50% |
| one or more Pt-containing catalysts | up to 0.50%, e.g. 0.03 to 0.50% |
| one or more diluents | 55.0 to 92.0% |

In a more specific variant, the coating composition comprises the following components in percent by weight:

| | |
|---|---|
| a vinyl-functionalized polysiloxane as a cross-linkable polysiloxane compound | 3.0 to 20.0% |
| one or more non-cross-linkable polysiloxane compounds | 8.0 to 25.0% |
| a copolymer having dimethylsiloxane and methylhydrosiloxane monomer units as a cross-linking polysiloxane compound, | 0.10 to 1.50% |
| one or more Pt-containing catalysts | up to 0.50%, e.g. 0.03 to 0.50% |
| one or more diluents | 55.0 to 92.0% |

In a more specific variant, the coating composition comprises the following components in percent by weight:

| | |
|---|---|
| a vinyl-functionalized polysiloxane as a cross-linkable polysiloxane compound | 7.5 to 15.0% |
| one or more non-cross-linkable polysiloxane compounds | 8.0 to 15.0% |

(continued)

| | |
|---|---|
| a copolymer having dimethylsiloxane and methylhydrosiloxane monomer units as a cross-linking polysiloxane compound, | 0.10 to 1.00% |
| one or more Pt-containing catalysts | up to 0.50%, e.g. 0.03 to 0.50% |
| one or more diluents | 55.0 to 82.0% |

In a more specific variant, the coating composition comprises the following components in percent by weight:

| | |
|---|---|
| a vinyl-functionalized polysiloxane as a cross-linkable polysiloxane compound | 7.5 to 15.0% |
| one or more non-cross-linkable polysiloxane compounds | 2.0 to 8.0% |
| a copolymer having dimethylsiloxane and methylhydrosiloxane monomer units as a cross-linking polysiloxane compound, | 0.10 to 1.00% |
| one or more Pt-containing catalysts | up to 0.50%, e.g. 0.03 to 0.50% |
| one or more diluents | 65.0 to 92.0% |

Optionally, the cross-linkable polysiloxane structural units, the low viscosity non-cross-linkable polysiloxane structural units, and/or the high viscosity non-cross-linkable polysiloxane structural units, may comprise dialkylsiloxane monomeric units, in particular dimethylsiloxane monomeric units. The cross-linkable polysiloxane structural units may be cross-linkable via one or more polymerizable end groups.

**Coated container**

In an embodiment, this disclosure relates to a coated container. The coating as described in detail herein is obtainable by the method described above. The coated container may be obtained by or obtainable by the method of this disclosure. The coated container comprises a hollow cylindrical body having a wall surrounding a lumen, the hollow cylindrical body having at least one opening, wherein at least a part of an inner surface of the wall comprises a coating.

An average coating thickness may be from 100 to 3000 nm.

Optionally, a total area of excessive coating thickness is less than 10% of the coating area and an excessive coating thickness is defined as an area exhibiting a coating thickness of more than twice, more than 1.50, or more than 1.20 the average coating thickness. In certain embodiments, the total area of excessive coating thickness is less than 7%, less than 5% or less than 3% of the coating area.

In an embodiment, the coating contains one or more silicon-organic polymers.

In certain useful embodiments, a total area of insufficient coating thickness is less than 5% of the coating area and an insufficient coating thickness is an area exhibiting a coating thickness of less than 100 nm or less than 50 nm. The total area of insufficient coating thickness may be less than 3% or less than 2% of the coating area.

The coated container of this disclosure may have a coating comprising one or more cross-linked polysiloxane structural units and one or more non-cross-linked polysiloxane structural units, wherein a ratio of a weight amount of cross-linked polysiloxane structural units and a weight amount of non-cross-linked polysiloxane structural units in the coating is less than 3.00, and optionally at least 0.40.

The coating may be disposed on an inner surface of the hollow cylindrical body of the container. The coating may have an average thickness of 100 nm or more, 200 nm or more, or 300 nm or more, 400 nm or more, or 450 nm or more. Optionally, the coating may have an average thickness of up to 3000 nm, up to 2000 nm, up to 1500 mm, up to 1000 mm, or up to 850 nm. A suitable coating thickness contributes to a tight seal at low temperatures. In embodiments, an average coating thickness may be from 100 nm to 3000 nm, from 200 nm to 2000 nm, or from 300 nm to 1500 nm, or from 400 nm to 1000 nm. Optionally, the indicated average coating thickness is present on at least 90%, at least 95% or at least 99% of the coated area. In embodiments, the average coating thickness is larger than 400 nm, particularly at least 450 nm or at least 500 nm. Exemplary preferred ranges for the average coating thickness are from >400 nm to 1500 nm, from 450 nm to 1250 nm, or from 450 nm to 850 nm.

In embodiments, the hollow cylindrical body described in this disclosure as part of a container may have an essentially constant inner diameter in order to allow for a stopper to expel essentially all of a composition present in the body. In this context, "essentially constant" includes inner diameter variations of not more than 200 $\mu$m, not more than 100 $\mu$m or not more than 50 $\mu$m.

The container may be any type of container, including a vial, a syringe, or a cartridge. In an embodiment, the

container is a syringe or cartridge.

The coating may contain one or more silicon-organic polymers. A "silicon-organic polymer" is a polymeric material comprised of monomeric units, the monomeric units comprising both silicon (Si) and carbon (C) atoms. An example of a silicon-organic polymer is a polysiloxane. In embodiments, the coating comprises one or more polysiloxane structural units. "Polysiloxane structural units" may refer to polysiloxane structures within a larger molecule (e.g. covalently bonded to a larger molecule, or part of a larger molecule) or to polysiloxane molecules per se. For example, cross-linked polysiloxane structural units are part of (covalently attached to) a polymeric network, whereas non-cross-linked polysiloxane structural units are present in the coating as molecules without being covalently attached to other molecules in the coating. Thus, the coating may comprise cross-linked polysiloxane structural units and/or non-cross-linked polysiloxane structural units. In this context, "cross-linked" means that the polysiloxane structural units are covalently attached to a polymeric network. Specifically, the term "cross-linked" includes the preferred case that a polysiloxane structure is covalently linked to other polysiloxane structures, e.g. through a polymeric backbone. Optionally, the cross-linked polysiloxane structural units are covalently bonded to other polysiloxane structures as a result of a hydrosilylation reaction. The polymeric backbone may for example be formed by polymerizing a polysiloxane carrying a polymerizable functional group such as a vinyl group. In contrast, "non-cross-linked" means that the polysiloxane is not covalently linked to other polysiloxane structures through a polymeric backbone, or not covalently linked to other polysiloxanes of the coating at all.

In an embodiment, the cross-linked polysiloxane structural units are cross-linked via one or more, e.g. two, end groups. The end groups may be selected from vinyl, acryl, methacryl, styrene, and combinations thereof. In an embodiment, the coating comprises a hydrosilylation reaction product of a cross-linkable polysiloxane compound and a cross-linking polysiloxane compound, such as a vinyl-polysiloxane compound and a polysiloxane having at least two Si-H groups. The cross-linking polysiloxane may cross-link the cross-linkable polysiloxane by reaction of its plurality of Si-H groups with vinyl groups of the cross-linkable polysiloxane. The reaction may be platinum catalyzed.

In this disclosure, "polysiloxane" or "polysiloxane structural unit" may refer to polyalkylsiloxane structural units, such as polydialkylsiloxane structural units. Optionally, one or more of the alkyl groups in the polyalkylsiloxane or polydialkylsiloxane are independently selected from branched or unbranched C1 to C8 alkyl groups. The alkyl groups may be linear alkyl groups. For example, the alkyl groups may be independently selected from methyl, ethyl, propyl, butyl, pentyl, hexyl, heptyl and octyl groups. For example, the alkyl groups may be independently selected from methyl and ethyl.

In an embodiment, the coating comprises both cross-linked polydialkylsiloxane structural units, and non-cross-linked polysiloxane structural units. Specifically, the coating may comprise cross-linked polydialkylsiloxane structural units, and non-cross-linked polysiloxane structural units, wherein the non-cross-linked polysiloxane structural units may be one or more silicone oils, i.e. polydialkylsiloxane structural units, such as polydimethylsiloxane silicone oil.

Optionally, a ratio of a weight amount of cross-linked polysiloxane structural units and a weight amount of non-cross-linked polysiloxane structural units in the coating is less than 3.00, less than 2.50, less than 1.80, or less than 1.20. The ratio of a weight amount of cross-linked polysiloxane structural units and a weight amount of non-cross-linked polysiloxane structural units in the coating may be at least 0.40, at least 0.60, or at least 0.70. In embodiments, this ratio ranges from 0.40 to 3.00, from 0.60 to 2.50, or from 0.70 to 1.80.

The coating may comprise more than one type of non-cross-linked polysiloxane structural units, such as at least two types, or at least three types. The types may differ in their viscosities. In some embodiments, the coating comprises high viscosity non-cross-linked polysiloxane structural units having a viscosity of more than 10,000 cSt, and/or low viscosity non-cross-linked polysiloxane structural units having a viscosity of 10,000 cSt, or less. Viscosity of the polysiloxane structural units may be determined according to DIN EN ISO 3219:1993 using a coaxial-cylinder system at 23°C and a shear rate of 10 $s^{-1}$. Optionally, the high viscosity non-cross-linked polysiloxane structural units have a viscosity of at least 15,000 cSt, and/or the low viscosity non-cross-linked polysiloxane structural units have a viscosity of 5,000 cSt, or less.

In an embodiment, the coating comprises the high viscosity non-cross-linked polysiloxane structural units but not necessarily the low viscosity non-cross-linked polysiloxane structural units.

A ratio of a weight amount of the low viscosity non-cross-linked polysiloxane structural units and the high viscosity non-cross-linked polysiloxane structural units ($mass_{high}$ : $mass_{low}$) may be at least 0.10, at least 0.50, at least 1.00, at least 1.50 or at least 2.00. In some embodiments, this ratio may range up to 5.00, up to 4.00 or up to 3.00. For example, the ratio of a weight amount of the low viscosity non-cross-linked polysiloxane structural units and the high viscosity non-cross-linked polysiloxane structural units may range from 0.10 to 5.00, from 0.50 to 4.00, or from 1.00 to 3.00.

The cross-linked polysiloxane structural units, the low viscosity non-cross-linked polysiloxane structural units, and/or the high viscosity non-cross-linked polysiloxane structural units, may comprise or consist of dialkylsiloxane monomeric units, in particular dimethylsiloxane monomeric units.

Optionally, the low viscosity non-cross-linked polysiloxane structural units have a weight average molecular weight of 1,200 to 30,000 g/mol, and/or the high viscosity non-cross-linked polysiloxane structural units have a weight average molecular weight of 15,000 to 300,000 g/mol. In an embodiment, the high viscosity non-cross-linked polysiloxane

structural units have a weight average molecular weight of 32,000 to 210,000 g/mol, or from 100,000 to 150,000 g/mol. In an embodiment, the low viscosity non-cross-linked polysiloxane structural units have a weight average molecular weight of 5,000 to 25,000 g/mol, or from 10,000 to 20,000 g/mol.

In embodiments, the low viscosity non-cross-linked polysiloxane structural units have a weight average molecular weight of at least 1,200 g/mol, at least 5,000 g/mol, or at least 10,000 g/mol. The weight average molecular weight may range up to 30,000 g/mol, up to 25,000 g/mol, or up to 20,000 g/mol.

In embodiments, the high viscosity non-cross-linked polysiloxane structural units have a weight average molecular weight of at least 15,000 g/mol, at least 32,000 g/mol, or at least 100,000 g/mol. The weight average molecular weight may range up to 300,000 g/mol, up to 210,000 g/mol, or up to 150,000 g/mol.

The coating may have a glass transition temperature at -60°C or below, optionally at -70°C or below, such as at -75°C or below, or at -80°C or below. Optionally, the glass transition temperature may be at -200°C or higher, at -150°C or higher, or at -120°C or higher, or at -100°C or higher. Glass transition temperature may be measured using differential scanning calorimetry (DSC), or thermomechanical analysis (TMA). An exemplary way of determining a coating's glass transition temperature includes thermomechanical analysis in expansion mode, for example using a Q400 thermomechanical analyzer by TA Instruments. The sample may be prepared by coating a container according to this disclosure, scraping off the coating using a scalpel and performing thermomechanical analysis in expansion mode, i.e. measuring the sample's expansion or contraction as a function of temperature. In embodiments, a glass transition temperature of the coating is in a range from -200 °C to -60 °C, from -150 °C to -70 °C, from -120 °C to -75 °C, or from -80°C to -100°C. In one embodiment, the glass transition temperature is from -80°C to -90°C.

The coating may be amorphous, or partially crystalline at room temperature. Optionally, the coating has a crystallinity of less than 20% (v/v) at 20°C.

In embodiments, the coating may have a crystallization temperature range and a melting temperature range determined using differential scanning calorimetry at temperature change rate of 10°C/min, wherein the crystallization temperature range and the melting temperature range overlap at a temperature of from -75°C to -100°C, particularly at -80°C. For example, DSC may be performed in a temperature range of -120 °C to -60°C. A suitable instrument is a DSC Q2000 (TA Instruments).

Without wishing to be bound by this theory, the inventors hypothesize that, within the range of overlap, both crystalline and molten portions of the coating exist. This is believed to give the coating good mechanical resistance. Crystallization temperature range and melting temperature range are considered to overlap, if the crystallization and melting peak areas extend into the same temperature range. For example, crystallization may start at -55 °C and end at -95 °C, i.e. an exothermal crystallization peak area may range from -55 °C to -95 °C; melting may start at - 90°C and end at -40°C, i.e. an endothermal melting peak area may range from -90 °C to -40 °C. In this example, the temperature range of overlap is -90 °C to -55 °C. This example fulfills the requirement of an overlap at a temperature from -75°C to -100°C because there is an overlap at at least one temperature within the indicated range.

Generally, this disclosure relates to containers for pharmaceutical compositions, comprising a hollow cylindrical body. The container may be configured for receiving a stopper that is slidable relative to the hollow cylindrical body from an open end towards an opposite end of the container. In an embodiment, the container has at least two open ends. One open end is such that a stopper can be inserted. Another open end may be on an opposite side of the container, e.g. in the case of a syringe, the side of the tip.

This disclosure is not particularly limited to container volumes. In an embodiment, the hollow cylindrical body encloses a volume of at least 0.10 ml, at least 0.50 ml, or at least 1.00 ml. Optionally, the volume may be up to 1,000 ml, up to 200 ml, up to 100 ml, or up to 25 ml. In embodiment, the volume ranges from 0.1 ml to 1,000 ml, from 0.50 ml to 200 ml, or from 1.00 ml to 25 ml. In an embodiment, the hollow cylindrical body encloses a volume of less than 10.0 ml.

The hollow cylindrical body has a lumen surrounded by a wall, wherein the wall may have a wall thickness of at least 0.50 mm, at least 0.80 mm, or at least 1.00 mm. Optionally, the wall thickness may range up to 10.0 mm, up to 8.0 mm, up to 5.0 mm, or up to 4.0 mm. In embodiments, wall thickness is from 0.50 to 10.0 mm, from 0.80 mm to 8.0 mm, or from 1.00 mm to 4.00 mm. The term "wall thickness" as used herein describes the shortest distance between an inner surface and an outer surface of the hollow cylindrical body.

The term "outer diameter" as used herein refers to the maximum distance between two points on the outer surface of the hollow cylindrical body, wherein the two points are connected by a straight line, which is perpendicular to and intersects with the longitudinal axis of the hollow cylindrical body. The term "inner diameter" as used herein refers to the maximum distance between two points on the inner surface of the hollow cylindrical body, wherein the two points are connected by a straight line, which is perpendicular to and intersects with the longitudinal axis of the hollow cylindrical body.

The hollow cylindrical body of the container may have an essentially constant inner diameter. This means that a total inner diameter variation is low. The "total inner diameter variation" is the difference between the largest inner diameter of a hollow cylindrical body and the smallest inner diameter of the same hollow cylindrical body. Optionally, the total inner diameter variation is less than 0.10 mm, less than 0.08 mm, less than 0.06 mm or less than 0.04 mm. The smaller the total inner diameter variation, the better. However, in some embodiments it may not be economically feasible to provide

containers with extremely small total inner diameter variations. Thus, in an embodiment, the total inner diameter variation may be at least 0.0001 mm, at least 0.001 mm, or at least 0.01 mm. For example, the total inner diameter variation may range from 0.0001 mm to <0.10 mm, from 0.001 mm to <0.08 mm, or from 0.01 mm to <0.04 mm.

The container according to aspects of this disclosure may have a standardized glide force of not more than 5.0 N. The glide force indicates the force needed to push a stopper in the hollow cylindrical body, whereas the break loose force indicates the forces needed to cause an initial movement of the stopper within the hollow cylindrical body. A "standardized glide force" is a glide force (GF) measured at standard conditions. Likewise, a "standardized break loose force" is a break loose force (BLF) measured at standard conditions. Standard conditions include a standard stopper, i.e. a Datwyler FM257/2 stopper made of bromobutyl rubber having a hardness of 52 Shore A, and density of 1.355 g/cm$^3$, available from Datwyler Pharma Packaging International NV, Industrieterrein Kolmen 1519, BE-3570 Alken, Belgium). BLF and GF can be measured in one take. A test for BLF and GF may be referred to as "BLGF" test. Any reference to a break loose force or glide force in this disclosure means the standardized break loose or glide force.

The standardized BLGF test is conducted on a universal testing machine at room temperature, i.e. 20 °C. A standardized BLGF testing device with a 50 N test cup is used for this purpose. The samples were fixed in vertical orientation in a universal testing machine model 106, 2 kN from TesT AG, CH-6331 Hünenberg, Switzerland.

The BLF is the force needed to move the stopper from its original position. The GF is the force needed to keep the stopper moving after breaking it loose.

The containers are filled with water for injection. After filling the specimen they are either stored or tested immediately, depending on the test purpose. The specimen are tested without needles.

The specimen are inserted into the holder and the pressure stamp is moved towards the stopper at a rate of 20 mm/min. Once a force of 0.25 N is measured the machine switches to the test rate of 100 mm/min and starts recording the data. The experiment ends when the measured force exceeds 35 N, which is usually the case when the distal end of the hollow cylindrical body is reached.

The BLF is the highest force measured within the first 4 mm of stopper movement. The GF values are measured within a test range starting after 4 mm of movement and ending 10 mm before reaching the distal end of the hollow cylindrical body, the GF according to this disclosure is the highest glide force measured in this experiment.

The containers of this disclosure may exhibit a standardized BLF of not more than 12.0 N. In some embodiments, the standardized BLF may be limited to upper limits of 9.0 N, 8.0 N, 7.0 N, 6.0 N, 5.0 N or even 4.0 N. The standardized BLF may be at least 0.1 N, at least 0.5 N, or at least 1.0 N so as to avoid any unintended movement of the stopper.

The container may exhibit a ratio of the standardized BLF relative to the standardized GF of BLF/GF >1.30. Optionally, the ratio of the standardized BLF relative to the standardized GF is characterized by BLF/GF ≤ 3.0. In embodiments, the ratio BLF/GF is >1.40, >1.50 or even >1.60 for the containers of this disclosure. In some embodiments, the ratio BLF/GF may be <2.5, <2.2, <2.0, or even <1.9.

The standardized GF of the containers of this disclosure may be <7.5 N, <6.5 N, <5.5 N, <4.5 N, <3.5 N, or even <2.5 N.

A suitable glide force and break loose force is relevant for a convenient use of containers of this disclosure. However, a sufficiently high break loose force may be beneficial for inhibiting undesired stopper movement during storage. Optionally, the container according to this disclosure has a standardized glide force of at least 0.5 N.

In order to safeguard a tight seal by inhibiting stopper movement during low temperature storage, containers of this disclosure may have a standardized break loose force that exceeds the container's standardized glide force by at least 30%, at least 60%, at least 100%, or at least 200%.

In an embodiment, this disclosure relates to a container as described herein, comprising a pharmaceutical composition. The pharmaceutical composition may comprise more than 60% by weight of water. Optionally, the pharmaceutical composition comprises a protein or nucleic acid therapeutically active agent.

## Examples

### Exemplary container

Referring now to the drawings, FIG. 1 illustrates a container 1 in an exemplary embodiment a syringe 3 for administering pharmaceuticals or cosmetics. The syringe 3 is made of a polymer and comprises a wall 5 surrounding a lumen. The container comprises a hollow cylindrical body 7, an opening 4 and a nestling surface 18 onto which, for example, an injection needle or a cap can be placed. A stopper 12 is inserted in the cylindrical portion and is slidable in the axial direction by pressure on a push rod 13. The syringe 3 has a flange 15 for handling purposes.

The container 1 is provided with a coating 10 on an inner surface, here specifically on the inner surface of the hollow cylindrical body 7. In this example, the coating 10 covers that region of the inner surface of the hollow cylindrical body 7 over which the stopper 12 can slide when the syringe is being emptied or used for drawing up.

## Coating compositions

Two exemplary coating compositions that are useful in the methods of this disclosure are shown in the table below.

| Component | Function | Composition A | Composition B |
|---|---|---|---|
| vinyl-terminated polydimethylsiloxane | cross-linkable polysiloxane structural units | 9.59 wt.% | 10.59 wt.% |
| methylhydrosiloxane/dimethylsiloxane copolymer | crosslinking polysiloxane | 0.24 wt.% | 0.26 wt.% |
| Pt complex | catalyst | 0.13 wt.% | 0.15 wt.% |
| silicone oil 20,000 cSt | non-cross-linkable polysiloxane structural units | 3.84 wt.% | 4.24 wt.% |
| silicone oil 1,000 cSt. | non-cross-linkable polysiloxane structural units | 9.46 wt.% | 0.00 wt.% |
| HMDSO | diluent | ad 100 wt.% | ad 100 wt.% |

## Coating thickness

Figure 2 illustrates the press-fit technique. An application body 20 is inserted in a pharmaceutical container 1, here a syringe, having an opening 4 and a hollow cylindrical body 7. A coating composition 8 is applied to the application body 20. The application body 20 is in direct physical contact with the inner surface of the container. The coating composition 8 is deposited in a deposition area of the inner surface while the application body 20 moves relative to the hollow cylindrical body 7, in this case it moves downward, leaving a layer of coating composition 8 on the inner surface. When the opening 4 of the container 1 is reached, the application body 20 is retracted from the container 1. A coated container 1 is obtained having a thickness distribution of the coating as illustrated in Figure 4A.

An application body 20 inserted in a hollow cylindrical body 7 with a wall 5 according to the press-fit technique is illustrated in Figure 3A, whereas Figure 3B shows an application body 20 inserted in a hollow cylindrical body 7 with a circumferential gap 22 between application body 20 and the inner surface of hollow cylindrical body 7. Application body 20 also comprises a duct 23 through which a coating composition can be applied to the application body such that it will flow downwards towards the equator of application body 20.

Figure 5A shows an exemplary application body 20 with an equator 24 and a hemispherical shape of the portion of application body 20 above equator 24, wherein $h_k$ is the height of the capillary bridge at the surface of the application body, and hw is the height of the capillary bridge at the inner surface of the hollow cylindrical body. Figure 5B shows a geometrical illustration of a capillary bridge 25 that may form between application body 20 and the inner surface. D denotes the size of the gap that the capillary bridge spans, $R_1$ is a radius of curvature of the application body, $\theta 1$ is the contact angle of the coating composition with the application body and $\theta 2$ with the inner surface of the hollow cylindrical body, I is the azimuthal radius, and r the meridonial radius, $\beta$ describes the position of the three-phase contact line on the particle surface. The height of the liquid meniscus is h.

A comparison experiment was performed, wherein a coating was applied by the press-fit technique, and another coating was applied using the capillary bridge technique of this disclosure, i.e. using the same method as illustrated in Figure 2, yet with an application body of smaller diameter such that a capillary bridge was formed. The coating composition and containers were the same for both methods. For this experiment, a TopPac® 1 ml lg LL syringe was used. The syringe is made of COC, having an inner surface with a surface energy of about 30 mN/m. The application body was a hemispherical PTFE head with a surface energy of about 18 mN/m. The coating composition had a surface energy of 13 mN/m and a viscosity of about 4 mPas. The syringe barrel had an inner diameter of 6.5 mm. The application body had a diameter at its equator of 6.4 mm.

Figure 4A shows a thickness analysis of a coating made according to the press-fit method. Figure 4B shows a thickness analysis of a coating made according to the method of this disclosure. It is apparent that the coating of Figure 4B is very homogeneous, with an average thickness of 730 nm. The total area of excessive coating thickness was less than 5% of the coating area. The total area of insufficient coating thickness (<100 nm) was less than 5% of the coating area.

The layer thickness distribution shown in Figure 4 was measured using RapID Layer Explorer measuring equipment. The Layer Explorer RapID is a computer assisted device with an integrated interferometer. A camera is also

connected for positioning. The RapID enables the measurement of average film thicknesses on the inner side of transparent, hollow cylindrical bodies, especially based on glass and plastic. Other cylindrical primary packaging materials, such as cartridges, can also be measured.

By means of the principle of white light reflectometry, a coating layer thickness is measured in a plurality of points (1 mm x 1 mm each) of the coating, such as at least 240 or at 480 points distributed over the coating area. When measuring several points on the coating, the coating distribution within the body can be determined quantitatively. The associated measurement mode is called BI mode (coating layer heights > 100 nm). The RapID can also be used to measure coating layer heights that are lower (> 20 nm). The measurements are made with a laser as light source. This measurement method is called as the UT mode (similar to ultra-thin). Coating layer heights are measured from 5 mm above the flange where any millimeter one point is measured up to the cone side. With these settings there are 49 measuring points along the barrel. After the entire length has been measured, the sample stage automatically rotates by a given angle and starts measuring a new line again. In this example, the degree of rotation of the sample stage was always kept at 30° which means 12 x 49 points = 588 points. Any image represented is based on 588 single measurements. The RapID software represents the data as a 2D false colour and converts them into a 3D elevation or height profile. The layer thickness of the coating is represented by grayscale (see Figure 4). Black colour indicates a layer thickness of 2000 nm, white indicates 0 nm or no measurement value. RapID analyser was used for layer thicknesses higher than 50 nm and counts any layer below 50 nm as non-existing. However, with special settings on the RapID device, layer thicknesses as low as 20 nm can also be measured.

## Claims

1. Method of making a coated container, comprising

    - providing a container comprising a hollow cylindrical body having a wall surrounding a lumen, the hollow cylindrical body having at least one opening,
    - inserting an application body into the lumen through the opening,
    - applying a coating composition to the application body such that the coating composition contacts at least a section of an inner surface of the wall,
    - depositing the coating composition on a deposition area of the inner surface of the wall by moving the application body relative to the hollow cylindrical body,
    - retracting the application body from the lumen through the opening,

    wherein the size of the application body is such that a circumferential gap is present between the application body and the inner surface of the wall during the depositing step.

2. Method according to claim 1, wherein the size of the circumferential gap is such that the coating composition forms a capillary bridge spanning the circumferential gap during the depositing step.

3. Method according to claim 1 or 2, wherein the size of the circumferential gap is **characterized by** a distance D of less than 0.10 mm between the application body and the deposition area during the depositing step.

4. Method according to claim 3, wherein the distance D is from 0.005 to 0.08 mm, or from 0.01 to 0.04 mm.

5. Method according to at least one of the preceding claims, wherein the wall comprises or consists of a polymeric material or glass.

6. Method according to at least one of the preceding claims, wherein the coating composition comprises in percent by weight:

| | |
|---|---|
| one or more cross-linkable polysiloxane compounds | 3.0 to 20.0% |
| one or more non-cross-linkable polysiloxane compounds | 2.0 to 25.0% |
| one or more cross-linking polysiloxane compounds | 0.10 to 1.50% |
| one or more catalysts | 0.03 to 0.50% |
| one or more diluents | 55.0 to 92.0% |

**7.** Method according to at least one of the preceding claims, wherein a rate of movement of the application body during depositing the coating composition is not more than a flow rate of the coating composition in the direction of movement of the application body.

**8.** Method according to at least one of the preceding claims, comprising the further steps of

- air flushing the container after depositing the coating composition on the deposition area, and/or
- curing the coating composition after depositing the coating composition on the deposition area to obtain a cured coating.

**9.** Method according to at least one of claims 3 to 8, wherein

- the cylindrical body has a total inner diameter variation in the deposition area of at most 2D, and/or
- the application body has a total outer diameter variation of at most 2D.

**10.** Method according to at least one of the preceding claims, wherein

- the application body comprises one or more ducts suitable for delivering the coating composition through the application body, and optionally wherein the step of applying a coating composition to the application body comprises delivering the coating composition through the one or more ducts, and/or
- applying a coating composition to the application body comprises delivering the coating composition to a section above the equator of the application body, allowing the coating composition to flow downwards on the application body; and/or
- applying a coating composition to the application body comprises delivering the coating composition to a section of the application body, and allowing the coating composition to build a capillary bridge between the application body and the inner surface of the wall.

**11.** Method according to at least one of claims 8 to 10, wherein after curing

- an average coating thickness is from 100 to 3000 nm, and/or
- a total area of excessive coating thickness is less than 10% of the coating area and an excessive coating thickness is defined as an area exhibiting a coating thickness of more than twice the average coating thickness.

**12.** Method according to at least one of claims 8 to 11, wherein after curing the coating contains one or more silicon-organic polymers.

**13.** Coated container, optionally obtained or obtainable by the method of one of the previous claims, comprising a hollow cylindrical body having a wall surrounding a lumen, the hollow cylindrical body having at least one opening, wherein at least a part of an inner surface of the wall comprises a coating, wherein

- an average coating thickness of from 100 to 3000 nm, and
- wherein a total area of excessive coating thickness is less than 10% of the coating area and an excessive coating thickness is defined as an area exhibiting a coating thickness of more than twice the average coating thickness.

**14.** Coated container according to claim 13, wherein the coating contains one or more silicon-organic polymers.

**15.** Coated container according to claim 13 or 14, wherein a total area of insufficient coating thickness is less than 5% of the coating area and an insufficient coating thickness is defined as an area exhibiting a coating thickness of less than 100 nm.

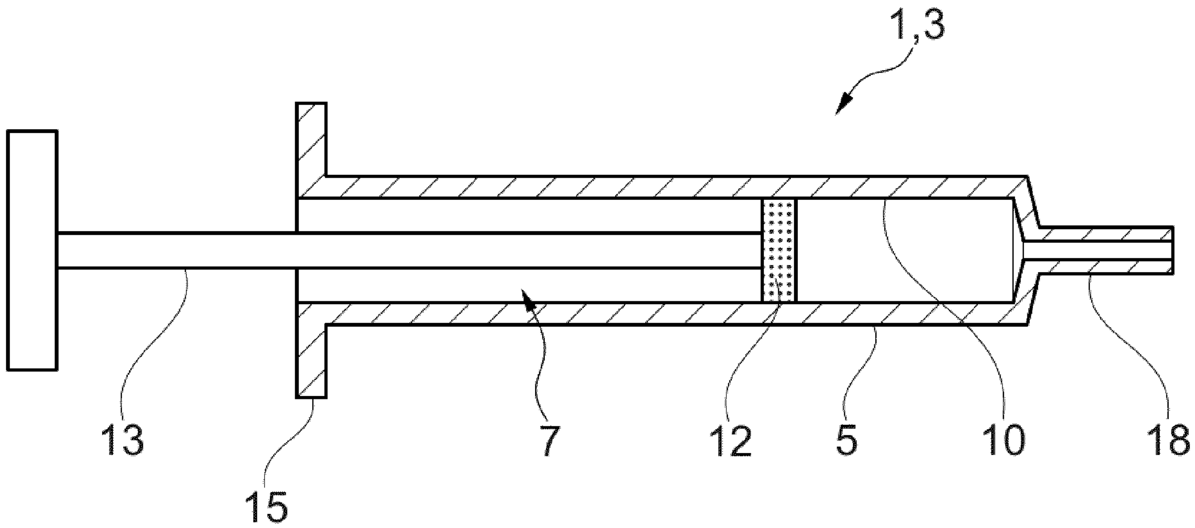


Fig. 1

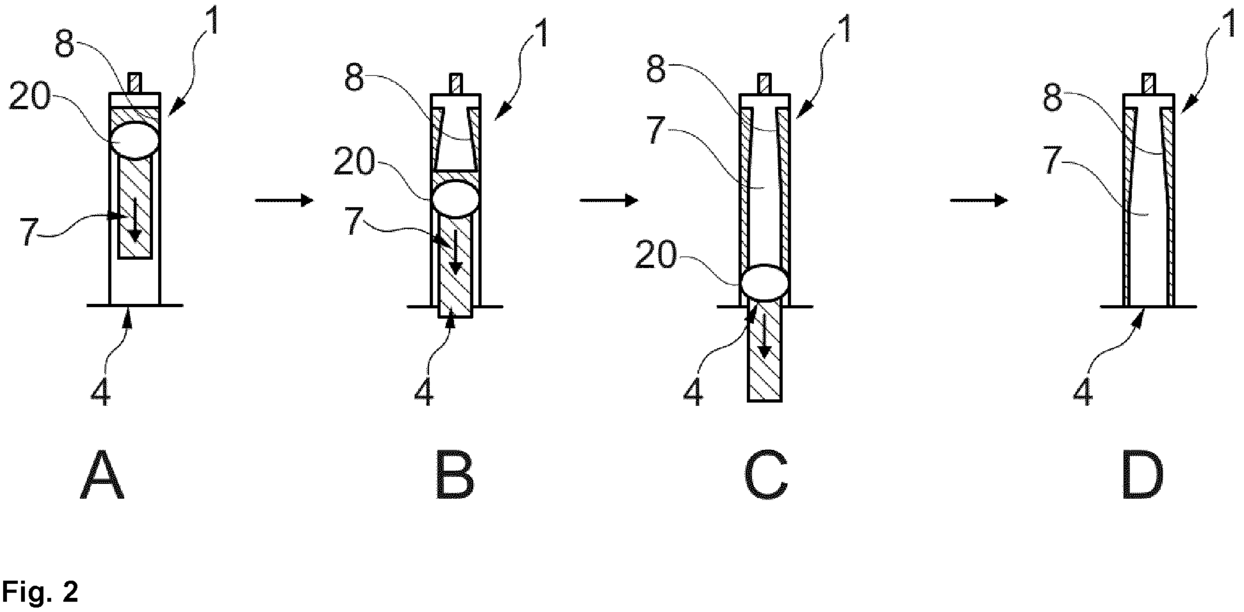


Fig. 2

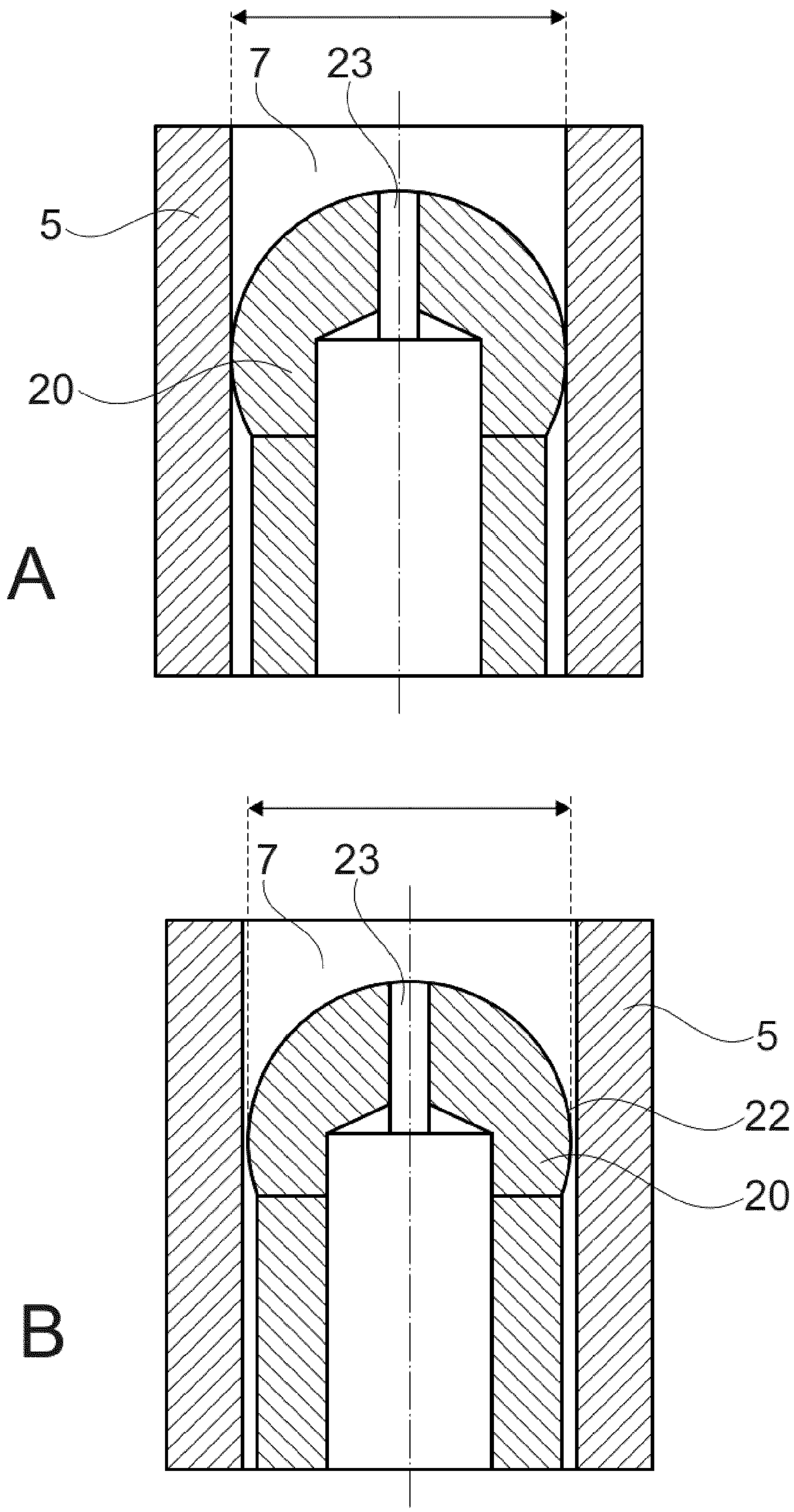


Fig. 3

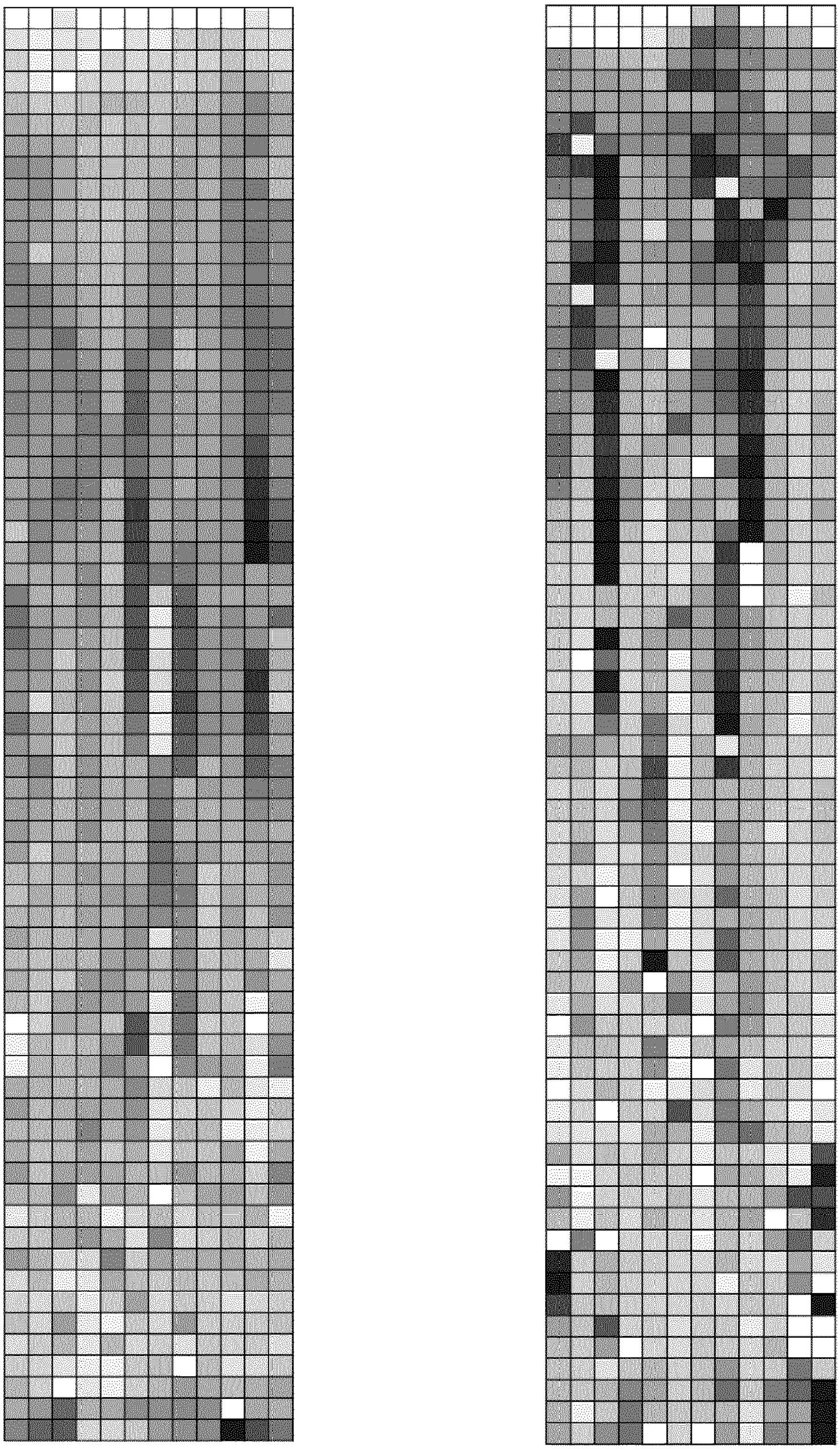

A B

Fig. 4

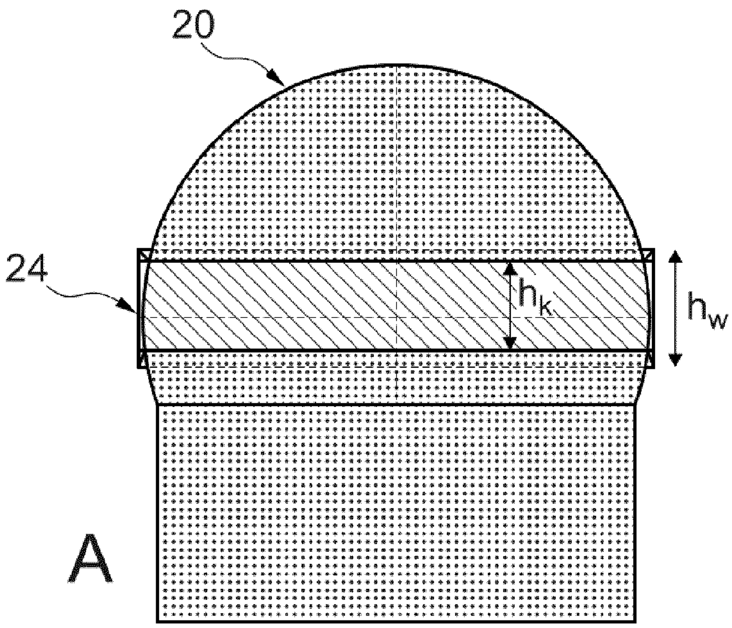


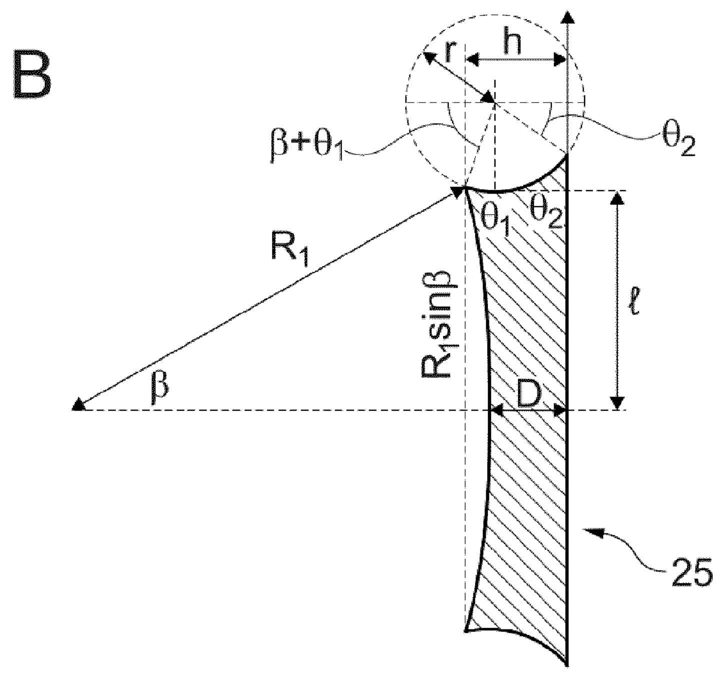


Fig. 5

Europäisches Patentamt
European Patent Office
Office européen des brevets

# EUROPEAN SEARCH REPORT

Application Number
EP 24 17 5732

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2002/012741 A1 (HEINZ JOCHEN [DE] ET AL) 31 January 2002 (2002-01-31)<br>* figures 1, 2 *<br>* paragraphs [0032] - [0051] *<br>* claims 1-10 *<br>----- | 1,5-15<br>2-4 | INV.<br>A61M5/31 |
| X<br>A | US 2023/218830 A1 (CHEVOLLEAU TZVETELINA [FR] ET AL) 13 July 2023 (2023-07-13)<br>* figures 1, 2 *<br>* paragraphs [0035] - [0053] *<br>----- | 1,5,8,12<br>2-4,6,7,9-11,13-15 | |
| X<br>A | US 2012/277686 A1 (MURAMATSU YASUHIRO [JP]) 1 November 2012 (2012-11-01)<br>* figures 2, 4 *<br>* paragraphs [0078], [0083], [0088], [0089] *<br>----- | 1,5<br>2-4,6-15 | |
| A | WO 2014/190225 A1 (SIO2 MEDICAL PRODUCTS INC [US]) 27 November 2014 (2014-11-27)<br>* figures 1-7 *<br>* paragraphs [0025] - [0044] *<br>----- | 1-15 | TECHNICAL FIELDS SEARCHED (IPC)<br>A61M |

The present search report has been drawn up for all claims

1

| Place of search | Date of completion of the search | Examiner |
|---|---|---|
| Munich | 30 October 2024 | López García, Mónica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 17 5732

This annex lists the patent family members relating to the patent documents cited in the above-mentioned European search report. The members are as contained in the European Patent Office EDP file on The European Patent Office is in no way liable for these particulars which are merely given for the purpose of information.

30-10-2024

| Patent document cited in search report | Publication date | Patent family member(s) | Publication date |
|---|---|---|---|
| US 2002012741 A1 | 31-01-2002 | AT E274943 T1 | 15-09-2004 |
| | | DE 10036832 C1 | 13-12-2001 |
| | | EP 1175918 A2 | 30-01-2002 |
| | | JP 3539409 B2 | 07-07-2004 |
| | | JP 2002143300 A | 21-05-2002 |
| | | US 2002012741 A1 | 31-01-2002 |
| US 2023218830 A1 | 13-07-2023 | CN 107596503 A | 19-01-2018 |
| | | CN 116870298 A | 13-10-2023 |
| | | EP 3481467 A1 | 15-05-2019 |
| | | JP 7042792 B2 | 28-03-2022 |
| | | JP 2019521781 A | 08-08-2019 |
| | | JP 2022046705 A | 23-03-2022 |
| | | KR 20190029637 A | 20-03-2019 |
| | | US 2019290856 A1 | 26-09-2019 |
| | | US 2023218830 A1 | 13-07-2023 |
| | | WO 2018011190 A1 | 18-01-2018 |
| US 2012277686 A1 | 01-11-2012 | EP 2495002 A1 | 05-09-2012 |
| | | JP 5602754 B2 | 08-10-2014 |
| | | JP WO2011052115 A1 | 14-03-2013 |
| | | US 2012277686 A1 | 01-11-2012 |
| | | WO 2011052115 A1 | 05-05-2011 |
| WO 2014190225 A1 | 27-11-2014 | NONE | |

EPO FORM P0459

For more details about this annex : see Official Journal of the European Patent Office, No. 12/82